# FASCICULE DE BREVET EUROPEEN

(11) **EP 0 328 839 B1**
(45) Date de publication et mention de la délivrance du brevet: **02.11.1994**
(21) Numéro de dépôt: 88403371.3
(22) Date de dépôt: 30.12.1988
(51) Int. Cl.: C12N 5/00

(54) **Tumeurs reconstituées, procédé d'obtention et kit de laboratoire pour sa mise en oeuvre**
Rekonstituierte Tumoren, Herstellungsverfahren und Zusammensetzung für ihre Anwendung
Reconstituted tumors; method of preparing it and laboratory kit for its use

(30) Priorité: 31.12.1987 FR 8718495
(43) Date de publication de la demande: 23.08.1989
(73) Titulaire: SANOFI, 75008 Paris (FR)
(72) Inventeur: Lugassy, Claire, F-75005 Paris (FR); Escande, Jean-Paul, F-92260 Fontenay-aux-Roses (FR)
(74) Mandataire: Gillard, Marie-Louise

(56) Documents cités:
- EP-A- 0 278 817
- WO-A-80/01350
- JOURNAL OF EXPERIMENTAL ZOOLOGY, vol. 232, no. 2, 1984, pp. 277- 285, New York, NY, US; E. BELL et al; p. 278
- LA RECHERCHE, vol. 18, no. 185, fevrier 1987, pp. 148-159, Paris, FR; L. DUBERTRET et al.; p. 152
- COMPTES RENDUS DE L'ACADEMIE DES SCIENCES, tome 305, serie III, 1987, pp. 507-513, Paris, FR; C. LUGASSY et al; p. 509

## Description

La présente invention a pour objet des tumeurs reconstituées en trois dimensions, un procédé d'obtention desdites tumeurs, un kit de laboratoire pour la mise en oeuvre du procédé et leur utilisation à des fins d'études cliniques ou d'essais pharmacologiques.

Le choix des molécules, pouvant servir de principe actif pour un médicament en vue du traitement de cellules différenciées malignes au sein d'un tissu, est facilité si l'on dispose de modèles d'étude sur lesquels une expérimentation in vitro peut être menée.

Il est possible de mettre en culture des fragments d'explants d'un tissu. Une première méthode de primo-culture (ci-après méthode classique) consiste à mettre les fragments à adhérer sur le fond plat d'un flacon de culture, immergés dans un milieu de culture, puis à les incuber jusqu'à ce qu'une sortie de cellules soit constatée. Une autre méthode de primo-culture d'explants de tissus récemment décrite (C. LUGASSY, O. ENJOLRAS et J.P. ESCANDE, C.R. Acad. Sc. Paris, T.301, Série III, n° 17, p. 779-784, 1985) - la méthode de culture sous lattice compacte couvrante - qui se caractérise par le fait que l'on recouvre les explants d'une lattice de derme-équivalent obtenue en rétractant du collagène par des fibroblastes, s'est révélée d'une grande efficacité.

Au-delà de la primo-culture, quelle que soit la technique mise en oeuvre, après un certain nombre de passages, on obtient en monocouche une population cellulaire homogène. Néanmoins, il est clair que chaque population ainsi obtenue a une organisation sans rapport avec celle d'un tissu où les cellules différenciées d'un type donné sont au contact avec les cellules du stroma et, en tout état de cause, parmi celles-ci des fibroblastes.

Il est d'abord apparu que seul un modèle à trois dimensions, associant cellules différenciées malignes du type soumis à expérimentation et fibroblastes, pourrait permettre de tester, dans des conditions expérimentales simulant le contexte rencontré in vivo, les molécules intéressantes pour le traitement d'un tissu lésé.

Il a déjà été montré (C. LUGASSY et J.P. ESCANDE, O.R. Acad. Sci. Paris, t.305, Série III, p. 507-513, 1987) qu'il était possible, par la reconstruction in vitro de mélanomes malins à partir des cellules recueillies lors de la culture sous lattice couvrante d'explants de mélanomes malins, d'obtenir de tels modèles à trois dimensions.

Ces modèles de l'art antérieur se caractérisent par le fait que les cellules différenciées y sont associées à des fibroblastes provenant de deux origines différentes et issus précisément les uns du stroma de l'explant du tissu primo-cultivé et les autres de la lattice elle-même.

La demanderesse a maintenant montré qu'il était possible de manière surprenante de préparer un modèle associant des cellules différenciées malignes d'un type donné, issues d'un explant de tumeur, et des fibroblastes d'un seul type.

L'invention concerne, selon un premier aspect, une tumeur reconstituée en trois dimensions, constituée de cellules différenciées malignes d'un type donné issues d'un explant d'une tumeur, de fibroblastes d'un seul type et de collagène.

Les cellules différenciées peuvent appartenir à tout type connu. Il s'agit, d'une manière préférée, de types cellulaires rencontrés, soit dans la moelle, soit dans le derme et dans ce cas plus particulièrement de mélanocytes. Ces cellules sont malignes. Elles peuvent être issues d'une tumeur primitive ou de métastases, préférentiellement de métastases. L'invention concerne notamment des tumeurs reconstituées en trois dimensions dont les cellules différenciées sont des mélanocytes issus d'un mélanome ou encore de blastes tumoraux.

Les cellules d'aspect fibroblastique peuvent provenir de diverses origines. Les fibroblastes d'origine vasculaire sont particulièrement bien adaptés. De préférence, on utilise des fibroblastes préparés à partir d'une tumeur vasculaire et, notamment, d'un angiome.

Selon l'invention, il est possible de construire à partir de cellules différenciées d'un type donné de très nombreuses tumeurs reconstituées, chacune associant ces cellules à un type de fibroblastes particulier L'ensemble de ces tumeurs reconstituées peut alors permettre, et c'est un avantage majeur de l'invention, une analyse comparée des inter-relations cellulaires dont sont capables les cellules différenciées au regard des fibroblastes ; cette analyse est notamment utile pour appréhender la croissance des tumeurs.

Les tumeurs reconstituées, selon l'invention, constituent des entités biologiques que l'on maintient en vie au contact d'un milieu de culture adapté à la culture et à l'entretien des cellules eucaryotes. Un milieu tel que le milieu RPMI 1640 (G.E. MOORE et al, J. Am. Med. Assoc. 199, 8(1967) 87-92), éventuellement complété, par exemple par un ou plusieurs acides aminés, du sérum de veau foetal et un ou plusieurs antibiotiques, convient.

L'invention concerne, selon un autre aspect, un procédé d'obtention des tumeurs reconstituées selon l'invention. Ce procédé se caractérise en ce que l'on prépare séparément, d'une part une population homogène de cellules différenciées malignes d'un type donné et d'autre part une population de fibroblastes, et que l'on met à incuber, en présence de collagène, ces deux populations.

Il convient de noter la simplicité de mise en oeuvre du procédé selon l'invention qui est remarquable, lorsque la population homogène des cellules dont on veut étudier la pathologie, peut être obtenue à partir d'une lignée de ces cellules. D'une manière générale, partant d'une tumeur, les cellules différenciées sont préparées à partir d'un explant de celle-ci. Des fragments de cet explant sont soumis à une primo-culture selon l'une des méthodes connues. Les cellules différenciées sortant de ces fragments sont recueillies, après un traitement à la trypsine tel que seules les cellules différenciées se détachent, et mises en culture. On procède alors à des repiquages successifs de manière à disposer in fine d'une suspension cellulaire dense.

Les fibroblastes sont préparés à partir de la culture d'un explant d'un tissu. L'explant, découpé en petits fragments, est mis en culture selon la méthode de primo-culture classique. Les fibroblastes contenus dans l'explant envahissent le flacon en 3 à 6 semaines ; on peut alors procéder à des repiquages successifs, en évitant généralement de dépasser 15 passages, de façon à disposer in fine d'une suspension cellulaire dense.

En ce qui concerne les cellules différenciées, il est possible de partir d'une lignée préétablie. Cette lignée est mise en culture et repiquée de manière à disposer in fine d'une suspension cellulaire dense.

Qu'il s'agisse des cellules différenciées ou des fibroblastes, il est bien entendu possible de conserver la suspension cellulaire finale ou toute suspension cellulaire intermédiaire sous forme congelée.

Un milieu de culture convenant pour la préparation des populations de cellules différenciées et de fibroblastes est également le milieu RPMI, éventuellement complété, notamment par un ou plusieurs acides aminés, du sérum de veau foetal et un ou plusieurs antibiotiques.

On met en présence, sous forme de suspension, les cellules différenciées et les fibroblastes. Leur mélange peut contenir par unité de volume 3/4 de cellules différenciées et 1/4 de fibroblastes ou inversement 1/4 de cellules différenciées et 3/4 de fibroblastes. Toutes les combinaisons intermédiaires sont possibles. Il convient seulement d'éviter que l'une des deux populations cellulaires n'ait un effectif inférieur au tiers de celui de l'autre population cellulaire. D'une manière préférée, on met en présence autant de cellules différenciées que de fibroblastes. Le nombre de cellules à introduire dépend, d'une part, du volume final dans lequel le mélange cellulaire va être mis à incuber après addition de collagène et, d'autre part, de la quantité de collagène introduite. Ce volume final est lui-même fonction de la taille du récipient à fond plat mis en oeuvre. A titre d'exemple, lorsque l'on utilise une boîte de Petri de 6 cm de diamètre, il est avantageux, en présence de 1,5 ml d'une solution de collagène à 1 mg/ml, pour un volume final de 4,7 ml, d'introduire globalement 2.10⁶ cellules sous un volume de 0,5 ml sous la forme d'un mélange préformé des deux catégories de cellules, contenant 4.10⁶ cellules par ml ou par addition de 0,25 ml d'une suspension d'une catégorie de cellules et de 0,25 ml d'une suspension de l'autre catégorie de cellules, chaque suspension contenant 4.10⁶ cellules par ml.

Le collagène utilisé est obtenu à partir de tissus conjonctifs ; les tendons de la queue du rat sont une source facile d'emploi. On prépare une solution de collagène légèrement acide, le collagène étant après extraction et purification mis en solution, par exemple, dans une solution à 1 pour 1000 (v/v) d'acide acétique. La solution de collagène mise en oeuvre peut contenir de 0,1 à 5 mg de collagène par ml. D'une manière préférée, elle contient 1 mg par ml.

A la solution de collagène placée dans un récipient à fond plat tel qu'une boîte de Petri, sont ajoutés, de préférence simultanément, les deux suspensions cellulaires ou leur mélange préalablement préparé, un milieu nutritif ainsi qu'un agent neutralisant tel que de la soude.

Le mélange obtenu est mis à incuber à une température adaptée à la culture de cellules de mammifères, et de préférence à 37°C. Après plusieurs jours, on constate l'apparition, sous l'action des cellules qui rétractent le collagène, d'un disque irrégulier dont les dimensions se stabilisent. Ce disque constitue une tumeur reconstituée à 3 dimensions selon l'invention.

Les dimensions de cette tumeur reconstituée sont fonction de la quantité de collagène et du nombre de cellules mises en jeu capables de rétracter le collagène. A titre d'exemple, lorsque l'on utilise 2.10⁶ cellules sous un volume de 0,5 ml pour 1,5 ml d'une solution de collagène à 1 mg/ml dans une boîte de Petri de 6 cm de diamètre, et en présence de 2 ml de milieu RPMI 1640 complété de 0,25 ml de NaOH et de 0,45 ml de sérum de veau foetal, on obtient, après 4 à 8 jours d'incubation à 37°C, une tumeur reconstituée dont la forme évoque celle d'un disque aux contours irréguliers de 5 à 10 cm de diamètre et de 0,5 à 2 mm d'épaisseur.

La tumeur reconstituée ainsi obtenue se caractérise notamment par son aptitude à produire, une fois transférée dans un flacon de culture et mise à adhérer sur le fond du flacon, d'une manière continue, des cellules qui s'échappent de sa périphérie dans l'ensemble de la boîte. On distingue des cellules d'aspect fibroblastique constituant un tapis et des cellules différenciées s'organisant en colonies à la surface de ce tapis ; elles sont issues les unes et les autres des cellules initiales mises en oeuvre.

Cette tumeur reconstituée constitue une entité biologique d'une grande stabilité en ce sens qu'elle conserve son organisation tissulaire et sa cohésion et produit en primo-culture des cellules des deux catégories initiales. Ce phénomène d'émergence cellulaire ne cesse que lorsque lesdites cellules sorties de la tumeur reconstituée viennent à confluence cellulaire et tapissent d'une manière complète le fond du récipient. Il est possible, néanmoins, de relancer ce phénomène d'émergence cellulaire en transférant la tumeur reconstituée dans un autre récipient neuf.

De manière surprenante, il est possible d'obtenir, à partir d'une tumeur reconstituée, obtenue à partir de cellules différenciées émergeant de fragments d'un explant d'une tumeur mis en primo-culture ou appartenant à une lignée cellulaire - dite tumeur reconstituée de première génération -, une deuxième tumeur reconstituée présentant des caractéristiques très voisines. De même, à partir de cette tumeur reconstituée - dite de deuxième génération - il est possible d'obtenir une tumeur reconstituée présentant des caractéristiques très voisines. D'une manière générale, il est en définitive possible, à partir d'une tumeur reconstituée de généra-d'ordre n, d'obtenir une tumeur reconstituée dérivée de génération d'ordre n+1 présentant des caractéristiques peu différentes de celles de la tumeur reconstituée de génération d'ordre n.

Il suffit, en effet, de recueillir les cellules sorties de la tumeur reconstituée initiale et de les mettre au contact d'une solution de collagène. La tumeur reconstituée dérivée est obtenue après une incubation de quelques jours. Une tumeur reconstituée d'ordre n+1 peut, comme il a été indiqué pour les tumeurs reconstituées de première génération, être transférée dans un milieu neuf.

Il a eté constaté, néanmoins, que, si une tumeur reconstituée d'ordre n+1 a des caractéristiques voisines de celles d'une tumeur reconstituée d'ordre n dont elle est issue, plus n est élevé, plus la tumeur d'ordre n+1 diffère de la tumeur reconstituée initialement obtenue d'ordre 1, en ce sens que, de génération en génération, les tumeurs reconstituées s'appauvrissent en cellules d'aspect fibroblastique et qu'en émerge en proportion un nombre toujours plus élevé de cellules différenciées, devenant incapables, en raison du nombre réduit de cellules d'aspect fibroblastique, de reconstituer - en prenant appui sur des cellules d'aspect fibroblastique ayant adhéré sur le fond du récipient - des colonies.

Les mêmes observations sont faites d'un transfert à l'autre d'une tumeur reconstituée donnée.

Cette évolution des tumeurs reconstituées selon l'invention, de génération en génération ou de transfert en transfert, n'est cependant pas un réel handicap pour l'utilisation desdites tumeurs reconstituées. Il est, en effet, toujours possible d'obtenir une tumeur reconstituée de première génération à partir des cellules différenciées émergeant d'une tumeur reconstituée quelconque selon l'invention. Il suffit de recueillir les cellules et de les mélanger selon le procédé de l'invention décrit ci-dessus, en présence de collagène à une suspension des fibroblastes préparés à partir de la population initialement mise en oeuvre pour l'obtention en primo-culture de la tumeur reconstituée d'ordre 1.

L'invention concerne, selon un autre aspect, un kit de laboratoire adapté à la mise en oeuvre du procédé d'obtention selon l'invention.

Ce kit est plus spécialement destiné aux laboratoires disposant de lignées tumorales et désirant les tester. Il se présente sous l'aspect d'une trousse. Il comporte du matériel de laboratoire et des réactifs permettant l'utilisation aux fins de l'invention d'une suspension congelée de fibroblastes conservée à -180°C par exemple dans l'azote liquide et dont l'efficacité a été testée. Cette suspension fait partie intégrante du kit.

D'une manière préférée, le kit contient le matériel et les réactifs en quantité suffisante pour la préparation de 5 à 10 lattices. Avantageusement, il contient notamment une solution de collagène à 1 mg par ml et de la soude 0,1M. Il peut aussi contenir des flacons à fond plat ainsi que des flacons utiles pour assurer le mélange des cellules différenciées et des fibroblastes en présence de collagène.

L'invention concerne selon un autre aspect, l'utilisation desdites tumeurs reconstituées en tant qu'agents d'études cliniques et outils de pharmacologie, lorsque se posent notamment le problème du pronostic d'une pathologie affectant des cellules différenciées et celui du choix des molécules pouvant servir de principe actif pour un médicament adapté au traitement de cette pathologie.

En effet, selon l'invention, il est possible, à partir des cellules différenciées malignes, de construire en laboratoire un grand nombre de tumeurs reconstituées, en associant à ces cellules soit les fibroblastes du stroma les accompagnant in situ, soit des fibroblastes d'une autre origine. Ces tumeurs reconstituées constituent alors des modèles expérimentaux en trois dimensions permettant de déterminer, tout au long de leur évolution suivie in vitro, différents paramètres biologiques dont l'analyse contribue, en fonction des observations antérieures, à en orienter l'étude clinique.

Dans les tumeurs reconstituées selon l'invention, les cellules malignes ont un comportement in vitro se rapprochant de leur comportement in vivo. Ce comportement se caractérise notamment par l'apparition de nodules et de foyers tridimensionnels de ces cellules émergeant d'un tapis de cellules fibroblastiques. On comprend qu'elles constituent les modèles d'une grande utilité en pharmacologie.

C'est ainsi qu'une molécule peut être testée pour son action anti-cancéreuse avec intérêt : la non-formation de nodules ou de foyers tridimensionnels ou un ralentissement de leur cinétique de formation permet de mesurer l'efficacité de la molécule testée. Une telle détermination qui ne pouvait être réalisée à l'aide de lignées tumorales servant de modèles d'étude constitue un progrès important.

### EXEMPLE 1

Cet exemple décrit l'obtention de trois tumeurs reconstituées à partir d'une lignée cellulaire de mélanocytes issus d'un mélanome malin et de fibroblastes de trois origines différentes, ainsi que l'obtention, à partir de ces trois mélanomes malins reconstitués (MMcoR1), de trois mélanomes malins de deuxième génération (MMcoR2) puis de trois mélanomes malins de troisième génération (MMcoR3). Parallèlement, un essai témoin est réalisé dans des conditions identiques, mais sans ajouter de fibroblastes. Une comparaison des caractéristiques des mélanomes malins reconstitués obtenus est présentée.

### 1 - REACTIF ET MATERIEL

**a) Collagène**
   Du collagène de type I a été extrait de queues de rats et préparé selon la technique connue (dont on trouvera une description dans Proc. Naty. Acad. Sci. USA, Vol. 76, n° 8, 1979, p. 1274), sous la forme d'une solution purifiée aqueuse à 1 pour 1000 (v/v) d'acide acétique contenant 1 mg de collagène par ml.
**b) Milieu de culture complet**
   Milieu RPMI 1640 (Gibco) additionné de :
   * 1 % de L-glutamine
   * 15 % (v/v) en final de sérum de veau foetal
   * pénicilline à raison de 100 unités par ml
   * streptomycine à raison de 100 µg par ml
**c) Flacons de culture**
   Des flacons dont une face plate présente une surface de 25 cm², commercialisés par la Société Falcon, ont été utilisés.

### 2 - MODE OPERATOIRE

### A) Obtention des mélanomes malins reconstitués de première génération

### a) Préparation d'une suspension de mélanocytes :

Des fragments d'un explant d'une métastase cutanée thoracique d'un mélanome malin humain du cou sont placés au contact d'une face plane d'un flacon (F1) de culture dans lequel 2 ml de milieu de culture complet ont été versés. Le flacon est porté à 37°C. Il est constaté à partir des fragments une émergence de cellules. Parmi ces cellules, dès la première semaine, on observe quelques cellules mélanocytaires au milieu de cellules d'aspect fusiforme. Après 5 à 8 jours, la quantité de milieu est portée à 5 ml. Le milieu est ensuite renouvelé tous les 5 jours.

Au terme de la quatrième semaine de l'incubation, les cellules d'aspect fusiforme recouvrent ladite face plane du flacon d'un tapis continu à la surface duquel apparaissent de nombreux îlots de mélanocytes. Le milieu de culture est alors éliminé par renversement du flacon, et de la solution de rinçage de HANKS (Proc. Soc. Exp. Biol. Med., 1949, 71, 196) est ajoutée puis, après un court temps de contact, éliminée.

L'addition de 0,5 ml d'une solution de trypsine à 0,25 % (p/v) dans le flacon permet alors, après un contact interrompu au bout de 3 minutes par l'addition de 5 ml de milieu complet, le détachement des seuls mélanocytes, dont l'adhérence est moins importante que celle des cellules fusiformes.

A l'aide d'une pipette, la suspension cellulaire est recueillie et centrifugée à 230 g pendant 5 min. Le culot - qui est pigmenté - est repris dans 10 ml de milieu complet. La suspension obtenue est répartie dans 2 flacons (F2) à raison de 5 ml par flacon. Les flacons sont mis à incuber 8 jours à 37°C en présence de 5 % de CO₂.

Les cellules de ce premier passage sont trypsinisées, recueillies, et repiquées comme indiqué ci-dessus dans 2 flacons (F3). Après 8 jours d'incubation à 37°C en présence de 5 % de CO₂, les cellules issues de ce deuxième passage sont trypsinisées. Les 4 suspensions cellulaires préparées à partir des 4 flacons (F3) sont regroupées et centrifugées à 230 g pendant 5 minutes. Le culot de ce dernier passage, qui est faiblement pigmenté, est mis en suspension et porté en incubation pour un troisième passage. A l'issue de ce dernier passage, les cellules sont recueillies après traitement par la trypsine, puis centrifugées. Le culot, qui est achromique, est remis en suspension dans un milieu complet en vue d'un comptage cellulaire dont il est tenu compte de manière à obtenir une suspension M1 contenant 4.10⁶ cellules par ml.

### b) Préparation des suspensions de fibroblastes

i) Origine des cellules
   Trois biopsies ont été pratiquées respectivement à partir
   * de la peau normale d'un sujet caucasien âgé de 24 ans.
      (ci-après fibroblastes a)
   * de la peau normale d'un sujet noir âgé de 28 ans.
      (ci-après fibroblastes b)
   * d'un angiome capillaire veineux de la face chez une femme de 20 ans.
      (ci-après fibroblastes c)
ii) Mode opératoire
   Chacun des 3 explants obtenus lors des biopsies est traité de la manière décrite au paragraphe ci-dessus. La confluence cellulaire est obtenue pour les primo-cultures en 3 à 5 semaines et pour les premier et second passages en 7 à 14 jours. Il est à noter qu'il est nécessaire de laisser agir la trypsine au moins 10 minutes pour obtenir le détachement des fibroblastes en raison de leur forte adhérence.

En definitive, chaque type de fibroblastes est obtenu sous la forme d'une suspension F1 contenant 4.10⁶ cellules par ml.

### c) Coincubation des mélanocytes et des fibroblastes en présence de collagène

On met en présence dans une boîte de Petri de 6 cm de diamètre contenant du milieu de culture les suspensions M1 et F1 en ajoutant du collagène, les différents composants et ingrédients étant apportés dans les proportions suivantes :

| | |
|---|---|
| - solution de collagène | 1,5 ml |
| - milieu de culture complet | 2 ml |
| - NaOH 0,1 M | 0,250 ml |
| - sérum de veau foetal | 0,450 ml |
| - suspension M1 | 0,25 ml |
| - suspension F1 | 0,25 ml |

On met donc en incubation 2.10⁶ cellules en présence de 1,5 ml d'une solution de collagène à 1 mg/ml.

Après 2 à 10 jours de rétraction à 37°C, il se forme une lattice qui est la reconstitution d'un mélanome malin dit de première génération (mélanome MMcoR1).

### B) Obtention des mélanomes malins co-reconstitués de deuxième génération et troisième génération

Après 8 à 15 jours d'incubation à 37°C, le mélanome MMcoR1 est transféré et mis à adhérer dans un flacon contenant du milieu de culture complet que l'on place en incubation à 37°C.

Le mélanome MMcoR1 donne naissance par migration hors de sa masse vers l'ensemble du fond du flacon à une population cellulaire P2, constituée également de cellules mélanocytaires différenciées et de cellules d'aspect fibroblastique. A partir de cette population P2, on prépare une suspension S2. 0,5 ml d'une solution de trypsine à 0,25 % (p/v) est ajouté. Après 5 à 15 minutes de contact, on constate le détachement et l'individualisation de l'ensemble des cellules. La suspension est recueillie par pipetage et ajustée de manière à contenir 4.10⁶ cellules par ml.

Un mélanome malin de deuxième génération (MMcoR2) est alors reconstitué en ajoutant, à 0,50 ml de la suspension S2, 1,5 ml de solution de collagène en présence de 2 ml de milieu de culture complet additionné de 0,25 ml de NaOH 0,1 M et de 0,45 ml de sérum de veau foetal.

D'une manière identique, à partir de la suspension S3 préparée à partir de la population P3, également constituée de cellules mélanocytaires différenciées et de cellules d'aspect fibroblastique, à laquelle donne issue le mélanome MMcoR2, un mélanome malin de troisième génération (MMcoR3) est reconstitué.

### C) Transfert des mélanomes malins reconstitués

Chaque mélanome malin reconstitué est mis en culture jusqu'à ce que les cellules, auxquelles il donne naissance dans le flacon, parviennent à confluence du fait de leur prolifération. Le mélanome est alors transféré et remis dans un flacon neuf contenant du milieu de culture complet. L'opération est renouvelée toutes les 4 à 8 semaines en fonction de la rapidité de la croissance cellulaire.

### D) Reconstitution de mélanomes malins de première génération à partir des trois mélanomes malins de troisième génération

0,5 ml d'une solution de trypsine à 0,25 % (p/v) est versé dans le flacon contenant le mélanome MMcoR3 obtenu en B. Après un contact de 5 minutes, la suspension cellulaire qui contient essentiellement des mélanocytes est prélevée, puis ajustée à 4.10⁶ cellules par ml. 0,25 ml de cette suspension est alors mélangé à 0,25 ml de la suspension F1 initialement utilisée pour l'obtention du mélanome de première génération selon le protocole décrit en 2c.

Trois nouveaux mélanomes malins ont ainsi été obtenus. Chacun présente les caractéristiques du MMcoR1 initial dont il est dérivé.

### 3 - RESULTATS

9 mélanomes reconstitués ont été obtenus : 3 de première génération (MMcoR1), 3 de deuxième génération (MMcoR2) et 3 de troisième génération (MMcoR3).

Le tableau ci-après présente leur origine et leur filiation :

| Cellules mises en oeuvre | Première génération | deuxième génération | troisième génération |
|---|---|---|---|
| mélanocytes+fibroblastes a | MMcoR1a | MMcoR2a | MMcoR3a |
| mélanocytes+fibroblastes b | MMcoR1b | MMcoR2b | MMcoR3b |
| mélanocytes+fibroblastes c | MMcoR1c | MMcoR2c | MMcoR3c |

Parallèlement, un essai témoin réalisé avec les seuls mélanocytes sans fibroblastes a conduit à l'obtention de trois générations successives de lattices de mélanocytes peu rétractées (LM) notées ci-après respectivement LM1, LM2 et LM3 et ne constituant pas des tumeurs reconstituées au sens de l'invention.

Pour chaque mélanome reconstitué et chaque lattice LM, on a noté son pouvoir de rétraction en mesurant son diamètre, sa capacité à adhérer sur le fond du récipient le contenant et la couleur du culot centrifugé après recueil des cellules migrant vers le milieu de culture afin d'apprécier la pigmentation cellulaire. Un examen microscopique de ces cellules a également été pratiqué.

Les observations suivantes ont éte faites :

### Pour la 1ère génération

### LM1

- Rétraction peu importante : partant d'un gel de 6 cm de diamètre, on obtient une lattice de 3 cm
- Adhérence nulle : la lattice transférée dans un flacon de culture se décolle au moindre mouvement
- Sortie de cellules : elle équivaut à la multiplication d'une lignée en passage
- Pigmentation : beige très clair.

### MMcoR1a et MMcoR1b

- L'évolution est la même pour ces 2 mélanomes
- Rétraction : 0,8 cm
- Adhérence : forte
- Sortie de cellules : apparition de colonies visibles à l'oeil nu de cellules tumorales et d'un tapis de cellules d'aspect fibroblastique
- Pigmentation : nulle dans les deux cas

### MMcoR1c

- Rétraction : 1,3 cm
- Adhérence : supérieure à celle de MMcoR1a et MMcoR1b ; le recueil de la population P2 (cf paragraphe 2B) a nécessité que l'on laisse agir la solution de trypsine pendant 15 min.
- Sortie de cellules : explosion de nodules tridimensionnels de cellules tumorales sur un fond de cellules d'aspect fibroblastique
- Pigmentation : nulle

### Pour la 2ème génération

### (en faisant référence aux observations précédentes)

### LM2

- Rétraction et adhérence semblables
- Pigmentation nulle
- Sortie de cellules : toujours aspect d'une lignée

### MMcoR2a et MMcoR2b

- Rétraction et adhérence un peu diminuées
- Pigmentation inchangée
- Sortie de cellules : diminution des cellules d'aspect fibroblastique qui persistent surtout autour de la lattice. Aspect progressivement proche d'une lignée en passage de cellules tumorales

### MMcoR2c

- Rétraction, adhérence et pigmentation inchangées
- Toujours présence de nodules tridimensionnels et de cellules d'aspect fibroblastique, mais d'apparition moins rapide

### Pour la 3ème génération

### (en faisant référence aux observations précédentes)

### LM3

- Evolution similaire avec ralentissement de la croissance cellulaire

### MMcoR3a et MMcoR3b

- Rétraction (2,2 cm) et adhérence diminuées, se rapprochant de celle de ACM3
- Sortie de cellules : raréfaction des cellules d'aspect fibroblastique et apparition d'une croissance de type lignée tumorale

### MMcoR3c

- Rétraction inchangée
- Adhérence un peu diminuée
- Sortie de cellules un peu ralentie, avec colonies visibles à l'oeil nu un peu moindre en nombre et en taille. Persistance des cellules fusiformes dont la croissance est ralentie aussi.

Il est important de noter qu'à partir de chacun des 3 mélanomes malins reconstitués de troisième génération (MMcoR3a, MMcoR3b et MMcoR3c) il a été possible, en réassociant les mélanocytes qui en sont issus aux fibroblastes de la suspension F1 initialement utilisée, d'obtenir un mélanome malin reconstitué dont les potentialités sont celles du mélanome malin reconstitué de première génération (soit respectivement MMcoR1a, MMcoR1b et MMcoR1c).

### EXEMPLE 2

Cet exemple décrit l'obtention d'une tumeur reconstituée à partir de blastes tumoraux issus de la moelle d'un patient atteint d'un lymphome de Burkitt.

### 1 - MODE OPERATOIRE

Une suspension de blastes tumoraux a été préparée à partir de cellules de la moelle du patient. Après incubation des cellules de la moelle dans un milieu de culture, on a observé la formation de petits foyers tridimensionnels constitués de blastes tumoraux sur un tapis de cellules fibroblastiques. Ces cellules ont été recueillies après trypsination et remises en suspension en milieu complet.

Cette suspension de blastes tumoraux a été coincubée avec une suspension de fibroblastes d'angiome (fibroblastes c de l'exemple 1) en présence de collagène.

Le mode opératoire mis en oeuvre, les réactifs et le matériel utilisés sont ceux décrits à l'exemple 1.

### 2 - RESULTAT

Après rétraction à 37°C, une tumeur s'est constituée. Après mise en culture de cette tumeur, la formation à son pourtour et à distance de foyers et de nodules de blastes tumoraux adhérant très fortement à un tapis de cellules fusiformes a été observée.

## Revendications

1. Tumeur reconstituée en trois dimensions, caractérisée en ce qu'elle est constituée de cellules différenciées malignes d'un type donné issues d'un explant d'une tumeur, de fibroblastes d'origine vasculaire et de collagène.

2. Tumeur reconstituée selon la revendication 1, caractérisée en ce que les cellules différenciées malignes sont issues d'une métastase de mélanome.

3. Tumeur reconstituée selon la revendication 2, caractérisée en ce que les cellules différenciées malignes sont issues de la moelle.

4. Tumeur reconstituée selon l'une quelconque des revendications 1 à 3, caractérisée en ce que les fibroblastes sont issus d'un angiome.

5. Procédé d'obtention d'une tumeur reconstituée, caractérisé en ce que l'on prépare séparément d'une part une population homogène de cellules différenciées malignes d'un type donné et d'autre part une population de fibroblastes d'origine vasculaire, et que l'on met en culture en présence de collagène ces deux populations.

6. Procédé d'obtention selon la revendication 5, caractérisé en ce que les cellules différenciées et les fibroblastes sont issus de deux explants différents.

7. Utilisation d'une suspension de fibroblastes d'origine vasculaire pour la mise en oeuvre du procédé d'obtention d'une tumeur reconstituée selon l'une des revendications 5 ou 6.

8. Tumeur reconstituée selon l'une quelconque des revendications 1 à 4, pour l'utilisation à des fins d'études cliniques ou d'essais pharmacologiques"

## Claims

1. Tumor reconstituted in three dimensions, characterized in that it is constituted of malignant differentiated cells of a given type issued from a tumor explant, of fibroblasts of vascular origin and of collagen.

2. Reconstituted tumor according to claim 1, characterized in that the malignant differentiated cells are issued from a melanoma metastases.

3. Reconstituted tumor according to claim 2, characterized in that the malignant differentiated cells are issued from the marrow.

4. Reconstituted tumor according to any one of claims 1 to 3, characterized in that the fibroblasts are issued from an angioma.

5. Method for obtaining a reconstituted tumor, characterized in that it consists in preparing separately, on the one hand a homogeneous population of malignant differentiated cells of a given type and on the other hand, a population of fibroblasts of vascular origin, and in placing both these populations in a culture medium in the presence of collagen.

6. Method according to claim 5, characterized in that the differentiated cells and the fibroblasts are issued from two different explants.

7. Use of a suspension of fibroblasts of vascular origin in carrying out the method for obtaining a reconstituted tumor according to one of claims 5 or 6.

8. Reconstituted tumor according to any one of claims 1 to 4, usable for the purpose of clinical studies or pharmacological tests.

## Patentansprüche

1. Rekonstituierter, dreidimensionaler Tumor, dadurch gekennzeichnet, daß er aus differenzierten, malignen Zellen eines gegebenen Typs besteht, die aus einem Explantat eines Tumors, Fibroplasten vaskulären Ursprungs und Kollagen stammen.

2. Rekonstituierter Tumor nach Anspruch 1, dadurch gekennzeichnet, daß die differenzierten, malignen Zellen aus einer Melanom-Metastase stammen.

3. Rekonstituierter Tumor nach Anspruch 2, dadurch gekennzeichnet, daß die differenzierten, malignen Zellen aus Mark stammen.

4. Rekonstituierter Tumor nach einem der Ansprüche 1 bis 3, dadurch gekennzeichnet, daß die Fibroplasten aus einem Angiom stammen.

5. Herstellungsverfahren für einen rekonstituierten Tumor, dadurch gekennzeichnet, daß man getrennt voneinander einerseits eine homogene Population differenzierter, maligner Zellen eines gegebenen Typs und andererseits eine Population aus Fibroplasten vaskulären Ursprungs herstellt und aus diesen beiden Populationen zusammen mit Kollagen eine Kultur anlegt.

6. Herstellungsverfahren nach Anspruch 5, dadurch gekennzeichnet, daß die differenzierten Zellen und Fibroplasten aus zwei verschiedenen Explantaten stammen.

7. Verwendung einer Suspension aus Fibroplasten vaskulären Ursprungs zur Anwendung des Herstellungsverfahrens für einen rekonstituierten Tumor nach einem der Ansprüche 5 oder 6.

8. Rekonstituierter Tumor nach einem der Ansprüche 1 bis 4 für die Verwendung zu klinischen Forschungszwecken oder pharmakologischen Versuchen.
